# EUROPÄISCHE PATENTSCHRIFT

(11) **EP 2 725 988 B1**
(45) Veröffentlichungstag und Bekanntmachung des Hinweises auf die Patenterteilung: **16.08.2017**
(21) Anmeldenummer: 12728393.5
(22) Anmeldetag: 04.06.2012
(51) Int. Cl.: A61B 17/34, A61B 17/29, A61B 1/00

(54) **TROKARSYSTEM**
TROCAR SYSTEM
SYSTÈME DE TROCART

(30) Priorität: 30.06.2011 DE 102011107613
(43) Veröffentlichungstag der Anmeldung: 07.05.2014
(73) Patentinhaber: Riek, Siegfried, 78628 Rottweil (DE); Bachmann, Karl-Heinz, 78667 Villingendorf (DE); Gaiselmann, Thomas, 78667 Villingendorf (DE)
(72) Erfinder: Riek, Siegfried, 78628 Rottweil (DE); Bachmann, Karl-Heinz, 78667 Villingendorf (DE); Gaiselmann, Thomas, 78667 Villingendorf (DE)
(74) Vertreter: Westphal, Mussgnug & Partner Patentanwälte mbB
(86) Internationale Anmeldenummer: PCT/EP2012/002354
(87) Internationale Veröffentlichungsnummer: WO 2013/000537

(56) Entgegenhaltungen:
- WO-A1-01/01847
- US-A1- 2008 147 002
- US-A1- 2009 163 768
- US-B1- 6 183 485

## Beschreibung

Die Erfindung betrifft ein Trokarsystem gemäß dem Oberbegriff des Patentanspruchs 1.

Trokarsysteme für die minimal-invasive Chirurgie bestehen in der Regel aus einem Trokardorn, um eine Öffnung in eine Körperhöhle (z.B. Bauchraum) zu schaffen, und einer Trokarhülse, die in dieser Öffnung verbleibt und einen Zugang zu dem Inneren der Körperhöhle für den operativen Eingriff bildet. Der Trokardorn weist eine distale Spitze auf, die zur Penetration des Körpergewebes, z.B. der Bauchdecke, dient, um die Öffnung zu schaffen. Die Spitze des Trokardorns kann dabei scharf, schneidend oder stumpf ausgebildet sein. Eine scharfe Spitze hat beispielsweise die Form einer dreikantigen Pyramide. Schneidende Spitzen weisen eine Klinge auf, die eine Gewebeinzision schafft, welche anschließend durch die konische Spitze dilatiert wird. Stumpfe Spitzen sind distal abgerundet, so dass sehr hohe Penetrationsdrucke für die Eröffnung der Gewebeschichten notwendig sind. Danach bewirken diese im Wesentlichen nur eine Dilatation einer zuvor erzeugten Läsion.

Bei diesen Trokaren, insbesondere den spitzen und schneidenden Trokaren besteht die Gefahr, dass bei der Penetration der Bauchdecke am Peritoneum anhaftende innere Organe, z.B. Darm bzw. Blutgefäße verletzt werden. Um dieses Risiko zu reduzieren werden sogenannte optische Trokare verwendet, wie sie beispielsweise in der US 5,685,820 A beschrieben sind. Bei diesen optischen Trokaren ist die distale Spitze als hohler durchsichtiger Kegel ausgebildet, der von innen durch eine Optik beobachtbar ist, welche in einem koaxial in dem Trokardorn verlaufenden Optikkanal aufgenommen ist. Der optische Trokar ermöglicht durch die durchsichtige Spitze eine dreidimensionale Sicht auf die Gewebeschichten der Bauchdecke, durch welche die Spitze des Trokars hindurchtritt. Dadurch bekommt der Operateur ein Gefühl für die Bewegung, die Geschwindigkeit und die Position der Trokarspitze bei der Penetration. Insbesondere kann vor der Penetration des Peritoneums erkannt werden, ob Adhäsionen zwischen Darm und Peritoneum an der Insertionsstelle vorhanden sind. Problematisch bleiben die hohen Penetrationsdrücke bei der Penetration der Fascie und des Peritoneums. Diese Penetrationsdrücke könnten zwar durch schneidende Klingen an der Trokarspitze reduziert werden, dies würde jedoch wiederum die Gefahr einer Verletzung des Darmes bei der Penetration erhöhen. Wird der bekannte optische Trokar mit oder ohne schneidende Kanten zur Penetration der Bauchdecke verwendet, so sind in jedem Falle relativ hohe Drücke und gegebenenfalls eine rotierende Bewegung des Trokars erforderlich. Dabei entsteht der sogenannte Tentingeffekt, bei welchem die Gewebeschichten, die einen hohen Penetrationsdruck erfordern, von der Trokarspitze zeltartig in das Abdomen gedrückt werden und sich dicht dem Retroperitoneum nähern können. Beim Eröffnen dieser Schichten geben diese dann plötzlich dem Penetrationsdruck nach und die Spitze dringt schlagartig in das Abdomen ein und der Operateur kann Schwierigkeiten haben, die Bewegung des Trokars zu kontrollieren, um nicht mit der Spitze des Trokars innere Organe oder große Gefäße des Retroperitoneums zu verletzen. Um dieses Problem zu reduzieren, werden von zahlreichen Operateuren optische Trokare mit stumpfer Spitze verwendet, wobei jedoch zunächst nach Art einer offenen Laparotomie eine Inzision der Bauchdecke erzeugt wird, die dann durch den stumpfen optischen Trokar nur noch dilatiert wird, ohne dass die Trokarspitze zur Penetration des Gewebes wirksam ist. Die offene Inzision der Bauchdecke widerspricht dabei allerdings der Zielsetzung der minimal-invasiven Operationstechnik.

Ein Trokarsystem gemäß dem Oberbegriff des Patentanspruchs 1 ist auch aus der WO 01/01847 A1 bekannt.

Die US 2009/0163768 A1 beschreibt ein System zum Einführen eines Instruments in das Körperinnere eines Patienten. Als Hilfsmittel kann dabei ein Endoskop verwendet werden, auf dessen distales Ende eine durchsichtige Spitze aufgesetzt ist. Die Spitze ist dabei als Fasszange ausgebildet, bei welcher die durchsichtige Spitze das erste Arbeitsteil bildet, während ein zweites Arbeitsteil gegen dieses erste Arbeitsteil beweglich an der Spitze gelagert ist. In einem vom proximalen Ende des Endoskops zu der distalen Spitze führenden Betätigungskanal ist ein Betätigungselement axial verschiebbar, dessen distales Ende an dem zweiten Arbeitsteil angreift, um dieses zu bewegen. Das Endoskop dient dazu, das Einführinstrument im Körperinneren unter Sicht erfassen zu können. Aus US 6,183,485 B1 ist ein Rohrschaft-Instrument bekannt, dessen distale Spitze als Fasszange ausgebildet ist. Das eine Arbeitsteil der Zange ist einstückig mit dem Rohrschaft ausgebildet, während das schwenkbar gelagerte zweite Arbeitsteil durch ein in dem Rohrschaft axial verschiebbar aufgenommenes Betätigungselement bewegbar ist.

Der Erfindung liegt die Aufgabe zugrunde, ein Trokarsystem zur Verfügung zu stellen, welches die Vorteile des optischen Trokars ausnützt und die Sicherheit bei der Gewebepenetration verbessert und eine Präparation im Gewebe erlaubt.

Diese Aufgabe wird erfindungsgemäß gelöst durch ein Trokarsystem mit den Merkmalen des Patentanspruchs 1.

Vorteilhafte Ausführungen der Erfindung sind in den Unteransprüchen angegeben.

Der wesentliche Gedanke der Erfindung besteht darin, den Trokar nicht nur als passives Werkzeug zu verwenden, welches manuell durch eine axiale Kraft und gegebenenfalls durch eine Rotationsbewegung durch die Gewebeschichten geführt wird. Der Trokar kann vielmehr erfindungsgemäß auch aktiv als Instrument verwendet werden, bei welchem die Gewebepenetration mittels der distalen Spitze erleichtert und sicherer gemacht wird und insbesondere auch eine Präparation im Gewebe mittels des Trokars möglich ist. Hierzu ist an der distalen Spitze des Trokardorns, die von innen optisch beobachtbar ist, das Arbeitsende eines Instruments ausgebildet, welches zwei gegeneinander bewegbare Arbeitsteile aufweist. Eines dieser Arbeitsteile ist einstückiger Bestandteil der Spitze, während das zweiten Arbeitsteil beweglich an der Spitze gelagert ist und mittels eines Betätigungselements gegenüber der Spitze und damit gegenüber dem ersten Arbeitsteil bewegbar ist. Das Betätigungselement ist axial bewegbar in einem Betätigungskanal geführt, der an dem des Trokardorn ausgebildet ist.

Beim Einstechen des Trokars zur Gewebepenetration kann das an der distalen Spitze angeordnete Arbeitsende bei Bedarf betätigt werden, um chirurgische Maßnahmen durchzuführen, die das Penetrieren der jeweiligen Gewebeschicht erleichtern oder die sich für eine sonstige operative Präparation eignen. Da das erste Arbeitsteil integraler Bestandteil der durchsichtigen distalen Spitze des Trokardorns ist, kann das Arbeitsende und dessen Funktion durch die Optik des optischen Trokars unmittelbar beobachtet werden, so dass der Operateur das Arbeitsende unter Sicht und damit mit minimalem Risiko betätigen kann. Auch das bewegliche zweite Arbeitsteil kann gegebenenfalls durchsichtig sein.

Das Arbeitsende kann in unterschiedlicher Form entsprechend den verschiedenen chirurgischen Instrumenten ausgebildet sein. In einer Ausführung ist das Arbeitsende zangenförmig mit zwei Backen ausgebildet. Die eine Backe ist durch die durchsichtige Wandung der distalen Spitze des Trokardorns gebildet, während die andere Backe durch das bewegliche zweite Arbeitsteil gebildet ist.

Das Arbeitsende des Instruments kann als Fasszange oder als Klemme ausgebildet sein. Weiter kann das Arbeitsende als Schere, als Dissektor oder als Koagulationsklemme ausgebildet sein. Für die Koagulation und/oder die Sektion ist eine bipolare Hochfrequenz-elektrische Ausführung möglich. Ebenso ist eine Ausführung mit hochfrequenten Ultraschallschwingungen möglich.

Bei einer Ausbildung als Fasszange kann bei der Gewebepenetration das an der distalen Spitze des Trokars anliegende Gewebe erfasst und festgehalten werden, so dass die jeweilige Gewebeschicht z. B. mittels eines Messers oder einer Schere durchtrennt und geöffnet werden kann. Ein solches Messer oder eine solche Schere können vorzugsweise durch einen zusätzlichen Arbeitskanal zur distalen Trokarspitze geführt werden. Bei einer Ausbildung des Arbeitselements als Koagulationsklemme und/oder Dissektor ist eine Präparation des Gewebes und gegebenenfalls eine Koagulation von Gefäßen mittels des Trokardorns selbst möglich. Die Verwendung des Trokars ist dadurch über die Schaffung eines Zugangs für die minimal-invasive Chirurgie hinaus erweitert, da auch operative Präparationen mittels des Trokarsystems durchgeführt werden können.

Ist das Arbeitsende zangenförmig ausgebildet, so ist das bewegliche Arbeitsteil vorzugsweise schwenkbar an der distalen Spitze des Trokardorns gelagert. Das erste Arbeitsteil ist vorzugsweise durch eine Abflachung oder eine Vertiefung in der Außenfläche der distalen Spitze gebildet. Im geschlossenen Zustand des Arbeitsendes legt sich das bewegliche Arbeitsteil in diese Abflachung oder Vertiefung, so dass sich das bewegliche Arbeitsteil vollständig in die Außenumfangskontur der distalen Spitze einfügt und die Gewebedilatation beim Eindringen der Trokarspitze nicht behindert.

In einer anderen Ausführung kann das zweite Arbeitsteil linear verschiebbar in der äußeren Mantelfläche der distalen Spitze des Trokardorns geführt sein. In diesem Fall kann das distale Ende des beweglichen zweiten Arbeitsteils mit einer in der Außenfläche der distalen Spitze ausgebildeten Anschlagkante zusammenwirken, um einen Stanz- oder Schneidvorgang zu ermöglichen. Hierbei ist das bewegliche Arbeitsteil vorzugsweise in einer Nut der äußeren Mantelfläche der distalen Spitze geführt, so dass sich auch hier das zweite Arbeitsteil in die Außenkontur der distalen Spitze einfügt.

Das bewegliche Arbeitsteil wird mittels des Betätigungselements betätigt, welches am proximalen Ende von dem Operateur gehandhabt wird oder gegebenenfalls an einen Ultraschallgenerator angeschlossen wird. Das Betätigungselement greift mit seinem distalen Ende an dem bewegbaren Arbeitsteil an. Um das bewegbare Arbeitsteil in beiden Richtungen zu betätigen, z. B. zum Öffnen und Schließen der Klemme, muss das Betätigungselement eine ausreichende Längssteifigkeit aufweisen. Das Betätigungselement kann an dem bewegbaren Arbeitsteil über ein punktförmiges Gelenk angreifen. Da sich bei der Bewegung des zweiten Arbeitsteils dieser Gelenkpunkt auf einer Bahn bewegt, die nicht exakt achsparallel verläuft, ist in dieser Ausführung das Betätigungselement an seinem distalen Ende elastisch aus der achsparallelen geraden Form auslenkbar. In einer anderen Ausführung greift das distale Ende des Betätigungselementes in eine zur Achsrichtung des Betätigungselements schräg gestellte Kulissenführung des zweiten Arbeitsteils ein.

Im Folgenden wird die Erfindung anhand eines in der Zeichnung dargestellten Ausführungsbeispiels näher erläutert. Es zeigen:
- Figur 1: einen Axialschnitt des distalen Endes des Trokardorns des Trokarsystems in geöffneter Stellung des Arbeitsendes,
- Figur 2: eine axiale Ansicht auf das distale Ende in der Position der Figur 1,
- Figur 3: einen Figur 1 entsprechenden Axialschnitt mit geschlossenem Arbeitsende,
- Figur 4: eine axiale Ansicht auf das distale Ende gemäß Figur 3 und
- Figur 5: in einem vergrößerten Teilausschnitt die Anlenkung des Betätigungselementes an das bewegliche Arbeitsteil.

Das Trokarsystem weist einen Trokardorn auf, der zur Penetration von Gewebeschichten, z. B. der Bauchdecke dient. Mittels des Trokardorns wird eine Öffnung zum Körperinneren, z. B. zur Bauchhöhle geschaffen. Das Trokarsystem kann zusätzlich eine Trokarhülse aufweisen, die mittels des Trokardorns in die Körperöffnung eingesetzt wird und nach dem Entfernen des Trokardorns als Zugang für einen minimalinvasiven Eingriff in der erzeugten Öffnung verbleibt.

In der Zeichnung und der nachfolgenden Beschreibung ist nur das distale Ende des Trokardorns dargestellt, an welchem die Erfindung verwirklicht ist. Die übrigen Teile des Trokarsystems entsprechen dem bekannten Stand der Technik, so dass sie nicht im Einzelnen beschrieben und erläutert werden müssen.

Der Trokardorn 10 des Trokarsystems hat die Form eines starren geraden Kreiszylinders aus Metall oder Kunststoff. Die distale Spitze 12 des Trokardorns ist im Wesentlichen kegelförmig ausgebildet und weist in der Regel eine stumpfe abgerundete Spitze auf. Die Mantelfläche kann auch leicht ballig ausgewölbt sein. Die Spitze 12 ist innen hohl und besteht aus einem durchsichtigen Kunststoff. Koaxial im Inneren des Trokardorns 10 verläuft ein Optikkanal 14, in welchen eine Optik 16, z. B. eine Stablinsenoptik oder ein Kamerachip gegebenenfalls auch mit integriertem Beleuchtungssystem eingesetzt werden kann. Die Optik 16 wird so eingesetzt, dass ihr distales Ende etwa in der Basisfläche der kegelförmigen Spitze 12 liegt. Mittels der Optik 16 kann die durchsichtige Spitze 12 des Trokardorns von innen ausgeleuchtet und beobachtet werden. Dadurch ist es für den Operateur möglich, dass außen an der durchsichtigen Spitze 12 anliegende Gewebe und das Vordringen der Spitze 12 durch das Gewebe zu beobachten. Insoweit entspricht das Trokarsystem und insbesondere der Trokardorn 10 dem aus dem Stand der Technik bekannten optischen Trokar.

Erfindungsgemäß ist an der distalen Spitze 12 des Trokardorns 10 das distale Arbeitsende eines chirurgischen Instruments ausgebildet. Dieses Arbeitsende besteht aus einem ersten Arbeitsteil 18 und einem mit diesem zusammenwirkenden zweiten Arbeitsteil 20. Das erste Arbeitsteil 18 ist.ein einstückiger Bestandteil der distalen Spitze 12 des Trokardorns 10. Dies bedeutet, dass das erste Arbeitsteil 18 in dem durchsichtigen Kunststoffmaterial der Spitze 12 des Trokardorns ausgeformt ist. Das zweite Arbeitsteil 20 ist außen an der distalen Spitze 12 in der Weise beweglich gelagert, dass das zweite Arbeitsteil 20 gegenüber dem ersten Arbeitsteil 18 bewegt werden kann. Auch das zweite Arbeitsteil 20 besteht vorzugsweise aus dem durchsichtigen Kunststoffmaterial.

Die Arbeitsteile 18 und 20 können im Wesentlichen in Form und Funktion den Arbeitsteilen bekannter chirurgischer Instrumente entsprechen. Dies bedeutet, dass die zusammenwirkenden Bereiche des ersten Arbeitsteils 18 und des zweiten Arbeitsteils 20 unterschiedliche Formen haben können. Im dargestellten Ausführungsbeispiel ist nur als Beispiel und nicht einschränkend das distale Arbeitsende in Form einer Klemme mit zwei gegeneinander bewegbaren Backen dargestellt. Ausführungen als Koagulationsklemme, als Dissektor usw. ergeben sich analog.

Im dargestellten Ausführungsbeispiel ist das erste Arbeitsteil 18 als eine in der äußeren Mantelfläche der Spitze 12 eingeformte Vertiefung ausgebildet. Die Vertiefung verläuft in einer Mantellinie der kegelförmigen Spitze 12 im Wesentlichen von der Basis der Spitze 12 bis zu deren distaler Kegelspitze. Der Grund der Vertiefung des ersten Arbeitsteiles 18 weist eine Riffelung 22 auf, die zum besseren Fassen und Greifen des Gewebes dient.

Das zweite Arbeitsteil 20 ist im dargestellten Ausführungsbeispiel als Backe ausgebildet, die mit ihrem proximalen Ende mittels eines Gelenkes 24 schwenkbar an dem Trokardorn 10 gelagert ist. Das Gelenk 24 befindet sich etwa am Übergang der distalen Spitze 12 in den zylindrischen Bereich des Trokardorns 10. Das zweite Arbeitsteil 20 erstreckt sich von dem Gelenk 24 bis zu dem distalen Scheitel der Spitze 12. Die Querschnittsform des zweiten Arbeitsteils 20 ist komplementär zu der Form der Vertiefung des ersten Arbeitsteils 18. An seiner in die Vertiefung des ersten Arbeitsteils 18 eingreifenden Kante weist das zweite Arbeitsteil ebenfalls eine Riffelung 26 auf.

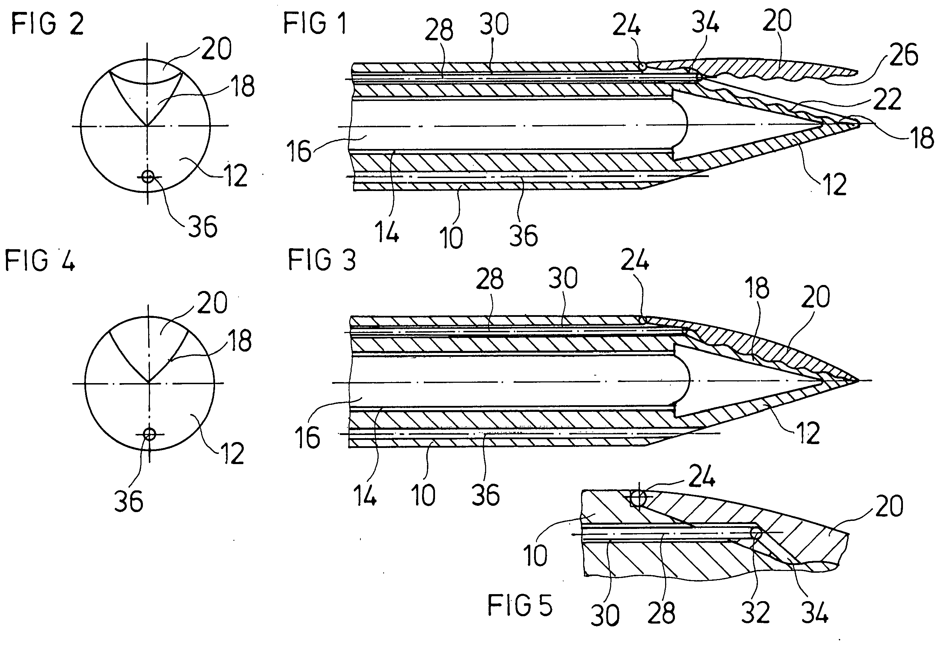

Das zweite Arbeitsteil 20 kann um das Gelenk 24 in eine in den Figuren 1 und 2 gezeigte Offenstellung geschwenkt werden. In dieser Offenstellung ist das zweite Arbeitsteil 20 aus der Vertiefung des ersten Arbeitsteils 18 herausgeschwenkt, so dass sich das erste Arbeitsteil 18 und das zweite Arbeitsteil 20 in distaler Richtung scherenförmig öffnen. Aus dieser Offenstellung kann das zweite Arbeitsteil 20 in die in den Figuren 3 und 4 gezeigte Schließstellung geschwenkt werden. In dieser Schließstellung fügt sich das zweite Arbeitsteil 20 formkongruent in die Vertiefung des ersten Arbeitsteils 18 ein. Dabei füllt das zweite Arbeitsteil 20 die Vertiefung des ersten Arbeitsteils 18 vollständig aus und die Außenfläche des zweiten Arbeitsteils 20 fügt sich vollständig in die kegelförmige Mantelfläche der Spitze 12 ein. Die Riffelung 22 des ersten Arbeitsteils 18 und die Riffelung 26 des zweiten Arbeitsteils 20 greifen hierbei ineinander.

Zum Verschwenken des zweiten Arbeitsteils 20 dient ein Betätigungselement 28. Das Betätigungselement 28 ist in einem Betätigungskanal 30 aufgenommen, der in der Wandung des Trokardorns 10 ausgebildet ist. Der Betätigungskanal 30 läuft achsparallel in dem Trokardorn 10 von dessen proximalem Ende bis zur distalen Spitze 12. Der Betätigungskanal 30 kann als Bohrung in der Wandung des Trokardorns 10 ausgebildet sein, wie dies im Ausführungsbeispiel gezeigt ist. Ebenso kann der Betätigungskanal 30 als Nut in der äußeren Mantelfläche des Trokardorns 10 ausgebildet sein. Das Betätigungselement 28 ist als dünner Stab ausgebildet, der axial verschiebbar in dem Betätigungskanal 30 geführt ist. Das Betätigungselement 28 ragt am nicht dargestellten proximalen Ende aus dem Betätigungskanal 30 und somit aus dem Trokardorn 10 heraus, so dass das Betätigungselement 28 durch den Operateur mittels geeigneter Griffelemente betätigt werden kann. Das distale Ende des Betätigungselements 28 greift an dem zweiten Arbeitsteil 20 an, um dieses um das Gelenk 24 zwischen der Offenstellung und der Schließstellung zu verschwenken. Um eine Schwenkbewegung in beiden Schwenkrichtungen aktiv zu ermöglichen, weist das Betätigungselement 28 eine ausreichende Längssteifigkeit auf.

In dem dargestellten Ausführungsbeispiel, wie es insbesondere aus Figur 5 ersichtlich ist, greift das Betätigungselement 28 mit einem an seinem distalen Ende ausgebildeten Querstift 32 in eine Kulissenführung 34 ein, die in dem zweiten Arbeitsteil 20 ausgebildet ist. Die Kulissenführung 34 ist als Schlitz ausgebildet, der zur Längsachse des Trokardorns schräg gestellt ist. Wird das Betätigungselement 28 in proximaler Richtung zurückgezogen, so läuft der Querstift 32 gegen das proximale Ende der Kulissenführung 34, wie dies in den Figuren 3 und 5 ersichtlich ist, wodurch das zweite Arbeitsteil 20 in seine Schließstellung gezogen wird. Wird das Betätigungselement 28 in distaler Richtung vorgeschoben, so wird der Querstift 32 in der Kulissenführung 34 in distaler Richtung verschoben und das zweite Arbeitsteil 20 wird in die in Figur 1 gezeigte Offenstellung gedrückt.

In einer alternativen Ausführung, die in der Zeichnung nicht dargestellt ist, kann das Betätigungselement 28 auch punktförmig an dem zweiten Arbeitsteil 20 angelenkt sein. Da der Anlenkungspunkt bei der Schwenkbewegung des zweiten Arbeitsteils 20 sich geringfügig auf einem Kreisbogen bewegt, weist das Betätigungselement 28 in dieser Ausführung eine geringe elastische Biegsamkeit auf, so dass das aus dem Betätigungskanal 30 austretende distale Ende des Betätigungselements 28 dieser kleinen Winkelbewegung folgen kann.

In dem in der Zeichnung dargestellten Ausführungsbeispiel ist das zweite Arbeitsteil 20 schwenkbar an der distalen Spitze 12 mit dem ersten Arbeitsteil 18 gelagert. In einer nicht dargestellten weiteren Ausführung kann das zweite Arbeitsteil auch in einer Mantellinie der Spitze 12 linear verschiebbar an der Außenseite der Spitze 12 gelagert sein. In diesem Falle ist das erste Arbeitsteil als Anschlagkante an der Außenseite der Spitze 12 ausgebildet. Mittels des Betätigungselements kann das zweite Arbeitsteil geführt linear in distaler Richtung verschoben werden, so dass sein distales Ende mit der Anschlagkante des ersten Arbeitsteils nach Art einer Stanze zusammenwirkt. Diese öffnet sich, wenn das zweite Arbeitsteil mittels des Betätigungselements in proximaler Richtung in die Offenstellung gezogen wird. In dem Trokarsystem ist außer dem Betätigungskanal 30 zumindest ein Arbeitskanal 36 ausgebildet, der vom proximalen Ende zum distalen Ende des Trokars führt. Im dargestellten Ausführungsbeispiel ist ein Arbeitskanal 36 diametral zu dem Betätigungskanal 30 angeordnet. Durch den Arbeitskanal 36 kann ein Miniaturinstrument hindurchgeführt werden, dessen Arbeitsende distal aus dem Arbeitskanal 36 an der distalen Spitze 12 herausragt. Das Miniaturinstrument kann beispielsweise eine Schere, ein Messer, ein Koagulationsinstrument, eine Miniaturkamera, ein Lichtleiter, ein Beleuchtungssystem, eine Optik oder dergleichen sein. Der Arbeitskanal 36 kann, wie in der Zeichnung dargestellt, in dem Trokardorn 10 ausgebildet sein. Alternativ kann der Arbeitskanal in einer den Trokardorn umschließenden Trokarhülse oder zwischen dem Trokardorn 10 und der Trokarhülse verlaufen. Solche Arbeitskanäle können nicht nur zur Einführung von Miniaturinstrumenten, sondern beispielsweise auch als Spülkanal dienen.

Wird bei der Gewebepenetration, z. B. bei der Penetration der Bauchdecke eine Gewebeschicht erreicht, die einen größeren Widerstand bietet, z. B. die Fascie oder das Peritoneum, so kann das Gewebe an der distalen Spitze 12 des Trokardorns 10 durch die Klemme aus den Arbeitsteilen 18 und 20 festgehalten werden und mittels eines durch den Arbeitskanal 36 eingeführten Miniaturinstruments eingeschnitten werden, um ein weiteres Eindringen der Trokarspitze zu ermöglichen.

Ist das Arbeitselement als Dissektor ausgebildet, so kann die Eröffnung der Gewebeschichten, z. B. der Facie oder des Peritoneums auch durch Präparation mittels des an der Spitze 12 des Trokardorns 10 ausgebildeten Arbeitsendes selbst durchgeführt werden. Ist das Arbeitsende durch elektrische oder hochfrequente Schwingungen betätigbar, so kann bei der Präparation auch gegebenenfalls eine Koagulation durchgeführt werden.

### Bezugszeichenliste

- 10: Trokardorn
- 12: Spitze
- 14: Optikkanal
- 16: Optik
- 18: erstes Arbeitsteil

- 20: zweites Arbeitsteil
- 22: Riffelung von 18
- 24: Gelenk
- 26: Riffelung von 20
- 28: Betätigungselement

- 30: Betätigungskanal
- 32: Querstift
- 34: Kulissenführung
- 36: Arbeitskanal

## Patentansprüche

1. Trokarsystem mit einem Trokardorn (10) mit einem koaxial in dem Trokardorn (10) verlaufenden Optikkanal (14) zur Aufnahme einer Optik (16) und mit einer hohlen durchsichtigen distalen Spitze (12) des Trokardorns (10), die mittels der Optik (16) von innen beobachtbar ist,
**dadurch gekennzeichnet, dass** an der distalen Spitze (12) das distale Arbeitsende eines Instruments ausgebildet ist, welches zwei gegeneinander bewegbare Arbeitsteile (18, 20) aufweist, dass ein erstes der Arbeitsteile (18) einstückiger Bestandteil der Spitze (12) ist und das zweite der Arbeitsteile (20) beweglich an der Spitze (12) gelagert ist, dass an dem Trokardorn (10) ein Betätigungskanal (30) ausgebildet ist, der von dem proximalen Ende des Trokardorns (10) an dessen distale Spitze (12) führt, dass in dem Betätigungskanal (30) ein Betätigungselement (28) axial verschiebbar aufgenommen ist, dessen proximales Ende aus dem proximalen Ende des Betätigungskanals (30) herausgeführt ist und dessen distales Ende an dem zweiten Arbeitsteil (20) angreift, um dieses zu bewegen, und dass das Trokarsystem zusätzlich zu dem Betätigungskanal (30) und dem Optikkanal (14) wenigstens einen axial durchgehenden Arbeitskanal (36) zum Durchführen eines Miniaturinstruments, einer Optik, eines Lichtleiters oder eines Beleuchtungssystems oder als Spülkanal aufweist.

2. Trokarsystem nach Anspruch 1,
**dadurch gekennzeichnet, dass** das erste Arbeitsteil (18) in die Wandung der distalen Spitze (12) des Trokardorns (10) eingeformt ist.

3. Trokarsystem nach Anspruch 1 oder 2,
**dadurch gekennzeichnet, dass** das zweite Arbeitsteil (20) zwischen einer Offenstellung und einer Schließstellung schwenkbar an der Spitze (12) des Trokardorns (10) gelagert ist.

4. Trokarsystem nach Anspruch 3,
**dadurch gekennzeichnet, dass** das erste Arbeitsteil (18) als Vertiefung in der Außenfläche der Spitze (12) ausgebildet ist und dass sich das zweite Arbeitsteil (20) in der Schließstellung komplementär in diese Vertiefung einfügt.

5. Trokarsystem nach Anspruch 1 oder 2,
**dadurch gekennzeichnet, dass** das zweite Arbeitsteil (20) in einer Mantellinie der Spitze (12) linear verschiebbar gelagert ist und mit dem als Anschlagkante in der Spitze (12) ausgebildeten ersten Arbeitsteil zusammenwirkt.

6. Trokarsystem nach einem der vorhergehenden Ansprüche,
**dadurch gekennzeichnet, dass** das Betätigungslement (28) längssteif ist.

7. Trokarsystem nach Anspruch 6,
**dadurch gekennzeichnet, dass** der Betätigungskanal (30) als achsparallele Bohrung in der Wandung des Trokardorns (10) oder als achsparallele Nut in der äußeren Mantelfläche des Trokardorns (10) ausgebildet ist und dass das Betätigungselement (28) stabförmig ausgebildet und axial verschiebbar in dem Betätigungskanal (30) geführt ist.

8. Trokarsystem nach einem der Ansprüche 1 bis 7,
**dadurch gekennzeichnet, dass** das Betätigungselement (28) mit seinem distalen Ende in eine zur Achse des Trokardorns (10) schräg gestellte Kulissenführung (34) des zweiten Arbeitsteils (20) eingreift.

9. Trokarsystem nach einem der Ansprüche 1 bis 7,
**dadurch gekennzeichnet, dass** das Betätigungselement (28) mit seinem distalen Ende punktförmig drehbar an dem zweiten Arbeitsteil (20) angelenkt ist.

10. Trokarsystem nach einem der vorhergehenden Ansprüche,
**dadurch gekennzeichnet, dass** das Arbeitsende des Instruments als Klemme, als Fasszange, als Schere, als Dissektor oder als Koagulationsklemme z.B. mit hochfrequentem Strom oder Ultraschall ausgebildet ist.

## Claims

1. Trocar system comprising a trocar (10)
having an optical channel (14) that extends coaxially inside the trocar (10) for receiving an optical unit (16), the trocar having a hollow transparent distal tip (12) of the trocar (10), that can be observed from the interior by the optical unit (16), **characterized in that** the distal working end of an instrument is configured at the distal tip (12) wherein the instrument includes two working parts (18, 20) which can be moved relative to each other, wherein a first of the working parts (18) is an integral component of the distal tip (12) and the second of the working parts (20) is movably supported on the distal tip (12), wherein an actuating channel (30) is configured on the trocar (10) and leads from the proximal end of the trocar (10) to its distal tip (12), **in that** an actuating element (28) is received in an axially displaceable manner inside the actuating channel (30), a proximal end being guided out from the proximal end of the actuating channel (30) and the distal end engaging on the second working part (20) in order to move this, and **in that** the trocar system, additionally to the actuating channel (30) and the optical channel (14), comprises at least one axially continuous working channel (36) for passing a miniature instrument, an optical unit, a fibre-optic light guide or an illumination system through or functioning as rinsing channel.

2. Trocar system according to claim 1, **characterized in that** the first working part (18) is integrated in the wall of the distal tip (12) of the trocar (10).

3. Trocar system according to claim 1 or 2, **characterized in that** the second working part (20) is pivotably supported at the distal tip (12) of the trocar (10) between an open position and a closed position.

4. Trocar system according to claim 3, **characterized in that** the first working part (18) is a depression in the exterior surface of the distal tip (12) and **in that**, in the closed position, the second working part (20) inserts itself in a complementary manner into this depression.

5. Trocar system according to claim 1 or 2, **characterized in that** the second working part (20) is supported in a linearly displaceable manner in a jacket line of the distal tip (12) and interacts with the first working part which is configured as a stop edge in the distal tip (12).

6. Trocar system according to any one of the above claims, **characterized in that** the actuating element (28) is longitudinally rigid.

7. Trocar system according to claim 6, **characterized in that** the actuating channel (30) is configured as an axially-parallel bore inside the wall of the trocar (10) or as an axially-parallel groove in the exterior jacket surface of the trocar (10) and **in that** the actuating element (28) is rod-like and guided axially displaceably in the actuating channel (30).

8. Trocar system according to any one of claims 1 to 7, **characterized in that** the actuating element (28) engages by the distal end thereof in a sliding bock guide (34) of the second working part (20) which is diagonally disposed relative to the axis of the trocar (10).

9. Trocar system according to any one of claims 1 to 7, **characterized in that** the actuating element (28) is rotatably articulated in a point arrangement by the distal end thereof to the second working part (20).

10. Trocar system according to any one of the above claims, **characterized in that** the working end of the instrument is configured as a clamp, as a pair of forceps, as a pair of scissors, as a dissector or as a coagulation forceps for example with high frequency current or ultrasound.

## Revendications

1. Système de trocart comprenant une broche de trocart (10) équipée d'un canal optique (14) s'étendant coaxialement dans cette broche de trocart (10) pour permettre de recevoir une optique (16) et d'une pointe distale (12) creuse transparente qui peut être observée de l'intérieur au moyen de l'optique (16),
**caractérisé en ce que**
sur la pointe distale (12) est formée l'extrémité distale d'un instrument qui comporte deux pièces de travail (18, 20) mobiles l'une par rapport à l'autre, une première des pièces de travail (18) est un composant intégré de la pointe (12) tandis que l'autre pièce de travail (20) est montée mobile sur la pointe (12), sur la broche de trocart (10) est formé un canal d'actionnement (30) allant de l'extrémité proximale de cette broche de trocart (10) à sa pointe distale (12), dans le canal d'actionnement (30) est logé un élément d'actionnement (28) mobile axialement dont l'extrémité proximale dépasse de l'extrémité proximale du canal d'actionnement (30) et dont l'extrémité distale vient en prise sur la seconde pièce de travail (20) pour permettre de la déplacer, et, le système de trocart comporte, en plus du canal d'actionnement (30) et du canal optique (14) au moins un canal de travail axial traversant (36) pour permettre le passage d'un instrument miniaturisé, d'une optique, d'un conducteur de lumière ou d'un système d'éclairage, ou constituant un canal de rinçage.

2. Système de trocart conforme à la revendication 1,
**caractérisé en ce que**
la première pièce de travail (18) est formée dans la paroi de la pointe distale (12) de la broche de trocart (10).

3. Système de trocart conforme à la revendication 1 et 2,
**caractérisé en ce que**
la seconde pièce de travail (20) est montée pivotante entre une position d'ouverture et une position de fermeture sur la pointe (12) de la broche de trocart (10).

4. Système de trocart conforme à la revendication 3,
**caractérisé en ce que**
la première pièce de travail (18) est réalisée sous la forme d'un évidement dans la surface externe de la pointe (12), et la seconde pièce de travail (20) s'insère de manière complémentaire dans cet évidement dans la position de fermeture.

5. Système de trocart conforme à la revendication 1 ou 2,
**caractérisé en ce que**
la seconde pièce de travail (20) est montée mobile linéairement dans la génératrice de la pointe (12), et coopère avec la première pièce de travail qui est réalisée sous la forme d'une arête formant butée dans la pointe (12).

6. Système de trocart conforme à l'une des revendications précédentes,
**caractérisé en ce que**
l'élément d'actionnement (28) est linéairement rigide.

7. Système de trocart conforme à la revendication 6,
**caractérisé en ce que**
le canal d'actionnement (30) est réalisé sous la forme d'un perçage parallèle à l'axe dans la paroi de la broche de trocart (10) ou sous la forme d'une rainure parallèle à l'axe dans la surface enveloppe externe de la broche de trocart (10), et l'élément d'actionnement (28) est réalisé sous la forme d'un barreau et est mobile axialement dans le canal d'actionnement (30).

8. Système de trocart conforme à l'une des revendications 1 à 7,
**caractérisé en ce que**
l'élément d'actionnement (28) vient en prise par son extrémité distale dans un guidage à coulisse (34) de la seconde pièce de travail (20) s'étendant obliquement par rapport à l'axe de la broche de trocart (10).

9. Système de trocart conforme à l'une des revendications 1 à 7,
**caractérisé en ce que**
l'élément d'actionnement (28) est articulé sur la seconde pièce de travail (20) par son extrémité distale en étant ponctuellement mobile en rotation.

10. Système de trocart conforme à l'une des revendications précédentes,
**caractérisé en ce que**
l'extrémité de travail de l'instrument est réalisée sous la forme d'une pince, d'une pince à fût, de ciseaux, de dissecteur ou de pinces de coagulation, par exemple, avec un courant haute-fréquence ou des ultrasons.
